Europäisches Patentamt

European Patent Office

Office européen des brevets

(19)

(11) **EP 1 320 625 B1**

(12) **EUROPÄISCHE PATENTSCHRIFT**

(45) Veröffentlichungstag und Bekanntmachung des Hinweises auf die Patenterteilung:
**14.09.2005 Patentblatt 2005/37**

(21) Anmeldenummer: **01974260.0**

(22) Anmeldetag: **17.09.2001**

(51) Int Cl.⁷: **C12P 9/00**
// C12P9:00, C12R1:38

(86) Internationale Anmeldenummer:
**PCT/EP2001/010729**

(87) Internationale Veröffentlichungsnummer:
**WO 2002/022842 (21.03.2002 Gazette 2002/12)**

(54) **POLYKONDENSATION VON ORGANISCHEN SILICIUMVERBINDUNGEN**

POLYCONDENSATION OF ORGANIC SILICON COMPOUNDS

POLYCONDENSATION DE COMPOSES ORGANIQUES DE SILICIUM

(84) Benannte Vertragsstaaten:
**AT BE CH CY DE DK ES FI FR GB GR IE IT LI LU MC NL PT SE TR**

(30) Priorität: **18.09.2000 DE 10046039**

(43) Veröffentlichungstag der Anmeldung:
**25.06.2003 Patentblatt 2003/26**

(73) Patentinhaber: **BASF Aktiengesellschaft**
**67056 Ludwigshafen (DE)**

(72) Erfinder: **FRIEDRICH, Thomas**
**63322 Rödermark (DE)**

(74) Vertreter: **Isenbruck, Günter**
**Isenbruck, Bösl, Hörschler, Wichmann, Huhn**
**Patentanwälte**
**Theodor-Heuss-Anlage 12**
**68165 Mannheim (DE)**

(56) Entgegenhaltungen:
• **DATABASE CA [Online] CHEMICAL ABSTRACTS SERVICE, COLUMBUS, OHIO, US; QIU, SHUYI ET AL: "Lipase -catalyzed bioconversion of organosilyl alcohol in microaqueous phase" retrieved from STN Database accession no. 128:229390 XP002187002 & HUANAN LIGONG DAXUE XUEBAO, ZIRAN KEXUEBAN (1997), 25(9), 51-54**
,

EP 1 320 625 B1

**Beschreibung**

Die Erfindung betrifft ein Verfahren zur Polykondensation von organischen Siliciumverbindungen in Gegenwart eines Enzyms.

Silicone und Silikate besitzen großtechnische Bedeutung. Silikate werden beispielsweise als Phasenmaterial in der Chromatographie eingesetzt. Es gibt zahlreiche Verfahren zu ihrer Herstellung. Verfahren, die zu amorphen Silikaten führen, gehen beispielsweise aus von Orthokieselsäure, welche unter Säure- oder Basenkatalyse in wäßriger Lösung kondensiert wird (A. F. Holleman, E. Wiberg, Lehrbuch der Anorganischen Chemie, Walter de Gruyter Verlag, Berlin New York 1985, 91. - 100. Auflage, S. 757 - 764). Silicone lassen sich durch Kondensation von Silanolen, Silandiolen und Silantriolen darstellen (A. F. Holleman, E. Wiberg, Lehrbuch der Anorganischen Chemie, Walter de Gruyter Verlag, Berlin New York 1985, 91. - 100. Auflage, S. 786 - 788). In den letzten Jahren wurden neue Verfahren entwickelt, bei denen organische Siliciumverbindungen bei milden Bedingungen kondensiert werden können. Als Katalysatoren werden Enzyme eingesetzt. Die Reaktionen lassen sich bei pH-Werten zwischen 6 und 8 durchführen. Ein geeignetes Enzym wurde 1998 erstmalig aus einem marinen Schwamm isoliert, wie in J. N. Cha, K. Shimizu, Y. Zhou, S. C. Christiansen, B. F. Chmelka, G. D. Stucky, D. E. Morse, Proc. Natl. Acad. Sci. USA 1999,96,361-365 beschrieben. Das Enzym ist aus drei Untereinheiten, den sog. Silicateinen, aufgebaut. Die Gewinnung des Enzyms ist relativ aufwendig. Neben der Polykondensation in Pufferlösung wurden die organischen Siliciumverbindungen $(EtO)_4Si$ und $(EtO)_3SiPh$ auch direkt mit luftgetrocknetem Enzym umgesetzt. WO 00/35993 beschreibt darüber hinaus den Einsatz von synthetischen Homopolymeren aus Cystein und Blockpolypeptiden aus Lysin und Cystein zur Polykondensation von Siliciumalkoxiden, Metallalkoxiden und deren Derivaten zu Silikaten, Polysiloxanen und Polymetalloxanen.

Aufgabe der vorliegenden Erfindung ist es, ein weiteres Verfahren zur Polykondensation von organischen Siliciumverbindungen zur Verfügung zu stellen, welches bei pH-Werten zwischen 6 und 8 durchführbar ist, sowie einen geeigneten Katalysator für diese Reaktion zu finden.

Die Lösung der Aufgabe geht aus von dem bekannten Verfahren zur Polykondensation von organischen Siliciumverbindungen in Lösung bei pH-Werten zwischen 6 und 8 in Gegenwart eines Enzyms. Das erfindungsgemäße Verfahren ist dadurch gekennzeichnet, daß als Enzym eine Lipase eingesetzt wird. Dies ist überraschend, weil Lipasen keinerlei strukturelle Ähnlichkeit zu bereits eingesetzten Enzymen wie Silicateinen aufweisen.

Für das erfindungsgemäße Verfahren sind im allgemeinen alle Lipasen geeignet, bevorzugt sind Lipasen aus Pseudomonas-Arten, besonders bevorzugt sind die Lipase aus *Burkholderia plantarii* (EC 3.1.1.3; SWISS-PROT: Q05489; L. G. J. Frenken, M. R. Egmond, A. M. Batenburg, J. W. Bos, C. Visser, C. T. Verrips, Appl. Environ. Microbiol. 1992, 58, 3787 - 3791), die Lipase A aus *Burkholderia cepacia* (EC 3.1.1.3; SWISS-PROT: P22088; S. Joergensen, K. W. Skov, B. Diderichsen, J. Bacteriol. 1991, 173, 559 - 567) und die Lipase A aus *Pseudomonas aeruginosa* (EC 3.1.1.3; SWISS-PROT: P26876; S. Wohlfarth, C. Hoesche, C. Strunk, U. K. Winkler, J. Gen. Microbiol. 1992, 138, 1325 - 1335), ganz besonders bevorzugt ist die Lipase aus *Burkholderia plantarii* geeignet.

Vorteilhaft ist, daß Lipasen über Fermentationsprozesse in großem Maßstab gewonnen werden können. Beispielsweise kann eine Lipase aus einem Bakterium dadurch gewonnen werden, daß das Bakterium, das die gewünschte Lipase sezerniert, in einem Nährmedium enthaltend Hefe-Extrakt, Sojaöl und übliche Zusatzstoffe wie Mineralsalze und Spurenelemente sowie gegebenenfalls Puffersubstanzen fermentiert wird. Nach Beendigung der Fermentation kann die Lipase von den Bakterienzellen und Zellbestandteilen z. B. durch Zentrifugation oder Filtration abgetrennt und durch Verfahren wie Ionenaustauschchromatographie, Molekularsiebchromatographie, hydrophobe Chromatographie und Fällungsmethoden gereinigt werden. Eine Reinigung der Lipase kann entfallen, wenn die Lipase nach Abtrennung von den Bakterienzellen und Zellbestandteilen an Trägern wie Polyolefinpartikeln und Polyurethanschaumstoffen immobilisiert wird. Immobilisierung bedeutet, daß Enzyme aus wasserfreien oder wäßrigen Lösungen insbesondere an unpolaren Matrices mit großer Oberfläche dauerhaft unter Beibehaltung der katalytischen Aktivität gebunden werden. Die Immobilisierung reduziert den Verlust an Lipase bei der Aufarbeitung und erlaubt die Durchführung der Polykondensation von organischen Siliciumverbindungen auch in organischen Lösungsmitteln, in denen freie Lipase nicht löslich ist. Zudem können die Polykondensationsprodukte auf dem Träger aufwachsen, so daß sich daraus neue Anwendungen in der Herstellung von Bauteilen in der Mikroelektronik ergeben können.

Werden Polyurethanschaumstoffe als Träger eingesetzt, werden die Enzyme auf dem Träger immobilisiert, indem die Enzyme mit reaktiven Gruppen an der Oberfläche des Polyurethanschaumstoffes reagieren. Die Enzyme werden kovalent an die Oberfläche gebunden. Solche reaktiven Gruppen können NCO-Gruppen, Epoxidgruppen, $CO_2H$-gruppen und/oder phenolische OH-Gruppen sein, die gegebenenfalls erst nach der Polymerisation an der Oberfläche des Polyurethanschaumstoffes angebracht wurden.

Werden Polyolefinpartikel als Träger eingesetzt, beruht die Bindung zwischen Enzym und Träger u.a. auf hydrophoben Wechselwirkungen. Als Polyolefine kommen Homo- und Copolymere aus gegebenenfalls substituierten Olefinen wie Ethylen, Propylen, Butadien, Buten, Octen oder Styrol in Betracht; bevorzugt wird Polypropylen als Träger eingesetzt.

Die Immobilisierung von Lipasen, die in durch Fermentation von Bakterien erhaltenen Lösungen, sog. Fermenterlösungen, vorliegen, auf Polyolefinpartikel wird im allgemeinen folgendermaßen durchgeführt: Die Bakterienzellen und Zellbestandteile werden aus der Fermenterlösung abgetrennt, z.B. durch Zentrifugation. Die zurückbleibende Lösung wird mit Wasser verdünnt, und die Polyolefinpartikel werden in Kontakt mit dieser Lipase enthaltenden Lösung gebracht. Das Inkontaktbringen erfolgt beispielsweise durch Zugabe der Polyolefinpartikel zur Lipase enthaltenden Lösung. Bei Inkontaktbringen der Lipase enthaltenden Lösung mit den Polyolefinpartikeln wird die Lipase an die Polyolefinpartikel adsorbiert. Die Polyolefinpartikel weisen eine sehr hohe Selektivität gegenüber Lipasen auf. Es werden überwiegend nur die Lipase und gegebenenfalls ihre Bruchstükke an die Polyolefinpartikel adsorbiert. Der Anteil an anderen Proteinen, der aus der Lipase enthaltenden Lösung an die Polyolefinpartikel adsorbiert wird, liegt im allgemeinen unter 2 Gew.-%. Die Adsorption stellt somit gleichzeitig einen Reinigungsschritt der Lipase von den weiteren Proteinen und Enzymen der Lipase enthaltenden Lösung dar.

Die Teilchengröße und der Hohlraumanteil der Polyolefinpartikel ist nicht kritisch. Bevorzugte Polyolefinpartikel weisen eine Teilchengröße von 100 μm bis 2000 μm, besonders bevorzugte Polyolefinpartikel weisen eine Teilchengröße von 200 μm bis 1000 μm auf. Der Hohlraumanteil der Polyolefinpartikel beträgt bevorzugt 40% bis 80%, besonders bevorzugt 60% bis 70%, ganz besonders bevorzugt 65%. Die Porengröße der Polyolefinpartikel beträgt vorzugsweise 0.01 μm bis 1 μm, besonders bevorzugt 0.05 bis 0.5 μm.

In einer Ausführungsform der Erfindung werden als Träger Polypropylenträger Accurel® der Firma Akzo mit den Korngrößen < 400 μm (Accurel 1004), 400 bis 1000 μm (Accurel 1001) und > 1000 μm (in Form von Pellets) eingesetzt, bevorzugt werden Accurel 1004 und Accurel 1001 eingesetzt.

Die optimale Dauer der Immobilisierung von Lipase auf Polyolefinpartikel hängt von der Lipase und der Art der Polyolefinpartikel ab und kann durch Routineversuche bestimmt werden. Im allgemeinen dauert die Immobilisierung 10 min bis 24 h, bevorzugt 4 bis 24 h, besonders bevorzugt 4 bis 6 h.

Im allgemeinen erfolgt die Immobilisierung bei einem pH-Wert zwischen 4.0 und 8.8, bevorzugt zwischen 4.5 und 7.8, besonders bevorzugt zwischen 4.5 und 6.0.

Die Ionenstärke, die durch Leitfähigkeitsmessung bestimmt werden kann, soll im allgemeinen < 0.5 M, bevorzugt < 0.3 M betragen.

Die Immobilisierung der Lipase auf den Polyolefinpartikeln wird auch als Beladung der Polyolefinpartikel mit Lipase bezeichnet. Eine bevorzugte Beladung, bei der möglichst viel Lipase adsorbiert wird und möglichst wenig Lipase in der Lösung verbleibt, ist von der Art des Polyolefins abhängig und kann über Routineversuche ermittelt werden. Im allgemeinen liegt die Menge an Lipase, die auf Polyolefinpartikeln immobilisiert wird, zwischen 0.1 und 50 mg Lipase pro g Träger, bevorzugt zwischen 0.5 und 20 mg Lipase pro g Träger, besonders bevorzugt zwischen 2 und 6 mg Lipase pro g Träger. In einer ganz besonders bevorzugten Ausführungsform der Erfindung liegt sie bei 4.2 mg Lipase pro g Träger.

Vorteilhafterweise wird die immobilisierte Lipase vor der Verwendung als Katalysator durch Waschen mit einem geeigneten Lösungsmittel wie Wasser von nicht adsorbiertem Material gereinigt. Anschließend wird sie gegebenenfalls an der Luft getrocknet. Die Restfeuchte beträgt im allgemeinen weniger als 4%.

Das beschriebene Verfahren zur Immobilisierung von Lipasen auf Polyolefinpartikel ist nicht nur mit Lipasen durchführbar, die in durch Fermentation erhaltenen Lösungen vorliegen, sondern generell mit Lipasen, die in wäßriger oder organischen Lösungsmitteln (beispielsweise in halogenierten oder nicht halogenierten aliphatischen und aromatischen Kohlenwasserstoffe) oder in wäßriger Pufferlösung vorliegen. Das Verfahren kann auch mit vor der Immobilisierung aufgereinigten Lipasen durchgeführt werden, eine Aufreinigung ist jedoch nicht nötig. Andere, dem Fachmann bekannte Verfahren zur Immobilisierung von Lipasen können ebenfalls angewendet werden.

Die immobilisierte Lipase kann ebenso wie die freie Lipase als Katalysator zur Polykondensation von organischen Siliciumverbindungen eingesetzt werden. Gegenüber der freien Lipase zeichnet sich immobilisierte Lipase durch eine erhöhte Stabilität und Standzeit unter kontinuierlicher und diskontinuierlicher Reaktionsführung sowie durch eine leichte Rückgewinnung der katalytisch aktiven Spezies bei diskontinuierlichen Reaktionsansätzen aus.

Für das erfindungsgemäßen Verfahren geeignete polykondensierbare organische Siliciumverbindungen sind

- $(RO)(R^1O)(R^2O)(R^3O)Si$, wobei R, $R^1$, $R^2$ und $R^3$ unabhängig voneinander $C_1$- bis $C_{10}$-Alkyl, $C_3$- bis $C_{10}$-Cycloalkyl, $C_4$- bis $C_{20}$-Alkylcycloalkyl, Aryl, $C_6$- bis $C_{16}$-Alkylaryl bedeuten können und die Alkylgruppen linear oder verzweigt sein können.

- $(RO)(R^1O)(R^2O)SiR^3$, wobei R, $R^1$, $R^2$ und $R^3$ unabhängig voneinander $C_1$- bis $C_{10}$-Alkyl, $C_3$- bis $C_{10}$-Cycloalkyl, $C_4$- bis $C_{20}$-Alkylcycloalkyl, Aryl, $C_6$- bis $C_{16}$-Alkylaryl bedeuten können und die Alkylgruppen linear oder verzweigt sein können.

- $(RO)(R^1O)Si(R^2)(R^3)$, wobei R, $R^1$, $R^2$ und $R^3$ unabhängig voneinander $C_1$- bis $C_{10}$-Alkyl, $C_3$- bis $C_{10}$-Cycloalkyl, $C_4$- bis $C_{20}$-Alkylcycloalkyl, Aryl, $C_6$- bis $C_{16}$-Alkylaryl bedeuten können und die Alkylgruppen linear oder verzweigt sein können.

- (RO)SiR$^1$R$^2$R$^3$, wobei R, R$^1$, R$^2$ und R$^3$ unabhängig voneinander C$_1$- bis C$_{10}$-Alkyl, C$_3$-bis C$_{10}$-Cycloalkyl, C$_4$- bis C$_{20}$-Alkylcycloalkyl, Aryl, C$_6$- bis C$_{16}$-Alkylaryl bedeuten können und die Alkylgruppen linear oder verzweigt sein können.

Bevorzugt ist der Einsatz von Tetraalkoxysilanen (RO)$_4$Si und Trialkoxyarylsilanen (R$^1$O)$_3$SiR$^2$ mit R, R$^1$ = Me, Et, Pr, iPr, Bu und R$^2$ = Aryl. Die Umsetzung von Silanen mit Arylgruppen bietet den Vorteil, daß die Arylgruppen Licht im UV-Bereich absorbieren und daher solche Silane mit UV-Detektoren nachweisbar sind.

Setzt man Tetraalkoxysilane und Trialkoxyalkylsilane zur Polykondensation ein, so können je nach Reaktionsführung lineare, verzweigte und vernetzte Silikate mit Hydroxy-, Alkoxy-, Alkyl-, Cycloalkyl-, Alkylcycloalkyl-, Aryl- und Alkylarylresten an der Oberfläche erhalten werden.

Die Polykondensation von Dialkoxydialkylsilanen führt zu Siliconen.

Setzt man Dialkoxydialkylsilane und Alkoxytrialkylsilane bei der Polykondensation von Tetraalkoxysilanen ein, so können diese als Regler dienen und die Molmasse der entstehenden Oligo- und Polysilikate reduzieren.

Wird freie oder immobilisierte Lipase eingesetzt, so kann als Lösungsmittel Wasser oder eine Pufferlösung eingesetzt werden, in denen die Lipase gut löslich ist. Die Pufferlösungen lassen sich durch Lösen von Puffersubstanzen in Wasser herstellen. Als Puffersubstanzen kommen als sog. Good'schen Puffer bekannte organische Puffersubstanzen in Betracht. Bevorzugt sind Puffersubstanzen, die Amino-Gruppen enthalten, besonders bevorzugt ist Trishydroxymethylaminomethan (sog. TRIS-Puffer). Weitere geeignete Puffersubstanzen, die in dem Bereich zwischen pH 6.5 und pH 7.4 puffern, können den Standard-Nachschlagewerken entnommen werden.

Die Ionenstärke der eingesetzten Pufferlösungen liegt im allgemeinen zwischen 1 mM und 100 mM, bevorzugt zwischen 5 mM und 50 mM, besonders bevorzugt zwischen 10 mM und 20 mM. Bei höheren Ionenstärken als 100 mM kann eine unkontrollierte Polykondensation der organischen Siliciumverbindungen ausgelöst werden. Beispielsweise können Phosphatpufferlösungen wie H$_2$PO$_4$$^-$/HPO$_4$$^{2-}$-Puffer, die im Bereich um pH 7 puffern, in einer Ionenstärke von über 100 mM mit den eingesetzten organischen Siliciumverbindungen reagieren.

Da die organischen Siliciumverbindungen in Wasser oder verdünnten wäßrigen Pufferlösungen schlecht löslich sind, ist eine gute Durchmischung der Reaktionslösung erforderlich.

Wird immobilisierte Lipase eingesetzt, so können auch organische Lösungsmittel wie höhere Alkohole mit bis zu acht C-Atomen, aliphatische und aromatische Kohlenwasserstoffe sowie Ether eingesetzt werden. Beispiele sind Methyl-tert-butylether, Cyclohexan und Cyclohexanol. Da die organischen Siliciumverbindungen in diesen Lösungsmitteln besser löslich sind als in Wasser, verläuft die Reaktion im allgemeinen in organischen Lösungsmitteln schneller als in wäßriger Lösung. Die Lösung in organischen Lösungsmitteln muß noch eine Restmenge an Wasser enthalten, die mindestens äquimolar den im Reaktionsansatz vorhandenen Alkoxygruppen ist, um eine Hydrolyse der Alkoxygruppen und damit eine Polykondensation zu ermöglichen.

Die Polykondensation wird im allgemeinen bei einem pH-Wert zwischen 6 und 8 durchgeführt, bevorzugt bei einem pH-Wert zwischen 6.5 und 7.4, besonders bevorzugt bei einem pH-Wert zwischen 6.8 und 7.2.

Im allgemeinen ist die Reaktion bei Temperaturen zwischen 0 und 60°C durchführbar, bevorzugt zwischen 10 und 40°C, besonders bevorzugt zwischen 20 und 30°C.

Die Reaktion kann sowohl bei Normaldruck als auch erhöhtem Druck von bis zu 2 bar durchgeführt werden, bevorzugt wird sie jedoch bei Normaldruck durchgeführt.

Die Reaktionsdauer beträgt im allgemeinen 1 h bis 24 h.

In einer Ausführungsform der Erfindung erfolgt die Reaktionsdurchführung durch Zugabe der organischen Siliciumverbindung zu der Lipase enthaltenden Lösung unter Rühren. In einer anderen Ausführungsform der Erfindung wird die organische Siliciumverbindung vorgelegt und die Lipase enthaltende Lösung unter Rühren zugeben. Die Zugabe der Reaktanden kann sowohl kontinuierlich als auch diskontinuierlich erfolgen, bevorzugt erfolgt sie diskontinuierlich.

Die organische Siliciumverbindung kann sowohl rein als auch in den oben genannten organischen Lösungsmitteln gelöst zugegeben werden.

Beim Einsatz von freier Lipase in Wasser und/oder Puffersubstanzen enthaltenden Lösungen entsteht im allgemeinen beim Zusammengeben von Lipase enthaltender Lösung und organischer Siliciumverbindung eine Emulsion. Wird immobilisierte Lipase eingesetzt, so entsteht eine Suspension. Das Produkt fällt meist in Form eines Niederschlages an.

Die Menge an eingesetzter Lipase wird angegeben in Units [U] pro ml Lösungsmittel. Die Aktivität der Lipase in wäßriger Lösung bei 20 bis 23°C wird durch Umsetzung des Substrats Tributyrin (= Glyceryltris(butanoat)) bestimmt, welches durch die Lipase in Glycerin und Buttersäure gespalten wird. Die Konzentration an Buttersäure läßt sich durch Titration mit NaOH (1N) bestimmen. Damit ergibt sich

$$1\ U = \text{Umsatz an Substrat } [\mu\text{mol}] \frac{1}{Zeit[\text{min}]}$$

Die Aktivität der Lipase ändert sich zwischen 15°C und 30°C nicht. 8.3 mg Lipase weisen beispielsweise eine Aktivität von 250000 Units auf.

Das Mengenverhältnis von freier Lipase, angegeben in Units pro ml $H_2O$, zu organischer Siliciumverbindung, angegeben in mol, liegt im allgemeinen zwischen 200 bis 5000 Units pro ml $H_2O$ und 4 mmol organischer Siliciumverbindung und bevorzugt zwischen 400 bis 2000 Units pro ml $H_2O$ und 4 mmol organischer Siliciumverbindung.

Das Mengenverhältnis von immobilisierter Lipase, angegeben in Units, zu organischer Siliciumverbindung, angegeben in mol, liegt zwischen 100 bis 20 000 Units pro 4 mmol organischer Siliciumverbindung, bevorzugt zwischen 100 bis 10 000 Units pro 4 mmol organischer Siliciumverbindung, in z.B. 1 ml $H_2O$ als Lösungsmittel.

Die Aufarbeitung erfolgt beim Einsatz von freier Lipase im allgemeinen durch Abtrennung der organisch-wäßrigen Phase und gegebenenfalls der organischen Phase von dem Produkt enthaltenden Niederschlag, bevorzugt durch Zentrifugieren. Anschließend wird der Niederschlag gegen ein organisches Lösungsmittel dialysiert. Als Lösungsmittel kommen alle mit Wasser mischbaren Alkohole wie beispielsweise Me-OH, EtOH, *i*PrOH und *n*PrOH in Betracht. Bevorzugt wird EtOH verwendet.

Die Abtrennung des Reaktionsproduktes kann auch durch Molekularsiebchromatographie, sowie - bei genügend hohen Molmassen - durch Zentrifugation, oder - bei genügend hydrophoben Reaktionsprodukten - durch Extraktion mit einem unpolaren Lösungsmittel erfolgen.

Die Lipase, die sich in der organisch-wäßrigen Phase befindet, kann zurückgewonnen werden, z.B. durch Ultrafiltration und Chromatographie.

Wird immobilisierte Lipase verwendet, so wachsen die Silicate auf dem Träger auf. Die Abtrennung des Reaktionsproduktes von der Lösung, in der die Reaktion stattfand, kann durch Filtration erfolgen.

Die Bestimmung der Molmasse des Produkts erfolgt in der Regel durch Gelpermeationschromatographie. Die Molmasse kann jedoch auch durch massenspektrometrische Methoden wie MALDI-MS (MALDI = matrix assisted laser desorption ionisation) oder ESI-MS (ESI = electronspray ionisation) bestimmt werden.

Die Erfindung wird durch die nachstehenden Beispiele näher erläutert.

**Beispiele:**

**Beispiel 1: Fermentation der Lipase aus** *Burkholderia plantarii*

Von einer Vorkultur, enthaltend 1 g $MgSO_4 \cdot 7H_2O$, 3,5 g $KH_2PO_4$, 3,5 g $K_2HPO_4$, 5,0 g $(NH_4)H_2PO_4$, 0,02 g $CaCl_2 \cdot 2H_2O$ und 10 g Hefe-Extrakt (Firma Difco) pro 1 l Wasser, wurden jeweils 200 ml in einem 1 l Schüttelkolben mit 2 Bodenschikanen für 30 min bei 121°C sterilisiert und mit üblichen Zusatzstoffen (Spurenelementen) versetzt.

Der Inhalt von zwei dieser Schüttelkolben, also 400 ml, wurde mit einer Ampulle *Burkholderia plantarii* 9 Stunden bei 30°C inkubiert, und so eine Vorkultur erhalten.

Ein Medium enthaltend 110 g Hefe-Extract (Difco), 38,5 g $K_2HPO_4$, 38,5 g $KH_2PO_4$, 55 g $(NH_4)H_2PO_4$, 11 g $MgSO_4 \cdot 7H_2O$, 0,22 g $CaCl_2 \cdot 2H_2O$, das auf 11 l mit vollentsaltztem

Wasser aufgefüllt wurde, wurde 30 min bei 121 °C sterilisiert und mit üblichen Zusatzstoffen (Spurenelementen) versetzt. Dieses Medium wurde anschließend in einen 14 l Fermenter gegeben.

Die Fermentation wurde bei 30°C, einem pH-Wert von 6,5 und einer Drehzahl von 1000 Umdrehungen pro Minute bei einer Begasung von 11 l $O_2$ pro min drei Tage lang durchgeführt. Während der Fermentation wurde kontinuierlich Sojaöl zugegeben. Die Zugabegeschwindigkeit wurde gemäß Formel I berechnet. Eine halbe Stunde nach Beginn der Reaktion wurde die Vorkultur zugegeben.

$$S = V * SF * (e^{(ST * t)} - 1)/t \qquad \text{(Formel I)}$$

S = Dosiermenge [g/h],
V = Fermentationsvolumen [1],
SF = 110 [g/lh],
ST = 0,01 [l/h],
t = Zeit seit Start [h]

Die Lipase wurde von dem Organismus in das umgebende Medium sezerniert. Nach Ende der Fermentation und Abkühlen auf 15 °C wurde die Lösung abgelassen. Die Lipase-Aktivität betrug 8900 U/ml, durch Zentrifugation konnte dieser Wert auf 10500 U/ml erhöht werden.

**Beispiel 2: Immobilisierung**

Die durch die oben beschriebene Fermentation erhaltene Fermenterlösung wurde bei 5000 bis 6000 Umdrehungen pro Minute in der swing-out Zentrifuge RC-3B in 1 l-Gefäßen abzentrifugiert, um die Zellen abzutrennen. Die Konzentration an Lipase betrug nun ca. 10 000 Units pro ml. 25 ml dieser Fermenterlösung wurden mit 75 ml Wasser verdünnt und mit 1 g Accurel 1001 inkubiert. Die Endkonzentration an Lipase betrug 250000 Units pro g Accurel 1001. Es wurden 8,3 mg Lipase auf 1 g Accurel immobilisiert. Dasselbe Ergebnis wurde mit Accurel 1004 erzielt.

**Beispiel 3: Polykondensation von organischen Siliciumverbindungen in Gegenwart von freier Lipase**

Zu 25 ml Wasser enthaltend 50 000 Units freie Lipase aus *Burkholderia plantarii* (wäßrige Phase) wurden bei Raumtemperatur und Normaldruck 25 ml Phe-

nyltriethoxysilan, Ph(EtO)$_3$Si, (organische Phase) gegeben und unter starkem Rühren vermischt, wobei eine Emulsion entstand, und sich Silikat in Form eines weißen Niederschlages in der organischen Phase bildete. Nach 24 h wurden die beiden Phasen durch Zentrifugation getrennt. Die überstehende Lösung der organischen Phase wurde abgetrennt, und der zurückbleibende weiße Niederschlag über Nacht gegen 2 l Ethanol dialysiert. Das Ausschlußvolumen der Dialysemembran betrug 10000 Da. Nach 24 h wurde die Probe dem Dialysesack entnommen und durch Zentrifugation ein unlöslicher Niederschlag abgetrennt. Der Niederschlag wurde durch GPC analysiert. Als Eichsubstanz wurde Polystyrol verwendet. Die Molmasse der Polymeren des entstandenen Polykondensationsgemisches lag zwischen 800 und 2400 Da. Demnach wurden Polymere hergestellt, die aus 4 bis 40 Einheiten aufgebaut sind.

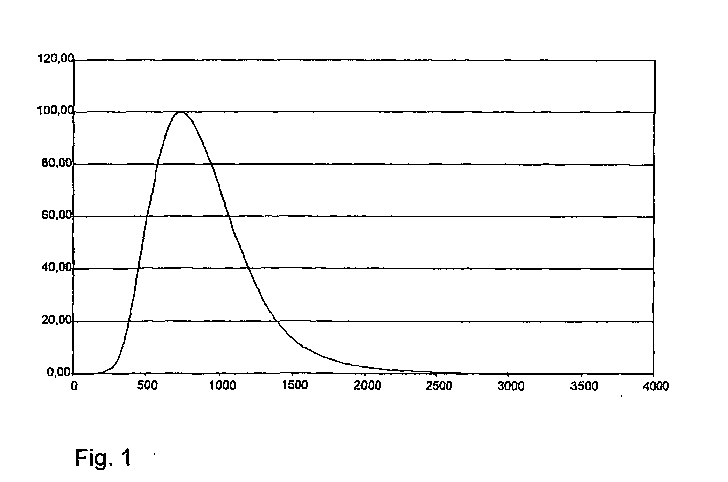

Figur 1 zeigt die Molmassenverteilung des Polykondensationsproduktes aus Beispiel 3.

### Beispiel 4: Bestimmung der Molmasse über Gelpermeationschromatographie

20 µL einer Lösung mit 22 mg/ml (Gesamtprobe aus Beispiel 3) wurden entnommen und über Sartorius Minisart SRP 25 (0,2 µm) filtriert. Zur GPC wurde eine S 628-Säule eingesetzt mit einem Säuleninnendurchmesser von 4,6 mm und einer Länge von 25 cm. Als Trennmaterial wurde PL gel Mixed B [5 µm] eingesetzt. Die Trennung erfolgte bei einer Säulen-Temperatur von 30°C mit THF als Elutionsmittel und einer Durchflußgeschwindigkeit von 0,3 mL/min. Bei dieser Durchflußgeschwindigkeit hatte die Säule eine Bodenzahl von 7000. Die Ausschlußgrenze für Polystyrol lag bei ca. $10^7$. Als Detektoren wurden ein UV-Photometer HP 1100 VWD bei 254 nm sowie ein Verdampfungs-Lichtstreudetektor ELS-1000 eingesetzt.
Die Kalibrierung erfolgte mit eng verteilten Polystyrol-Standards der Firma Polymer Laboratories mit Molmassen von 580 bis 7,5·$10^6$, sowie Hexylbenzol (Molmasse von 162). Ausserhalb dieses Intervalls liegende Elutionsbereiche wurden durch Extrapolation geschätzt. Die Ergebnisse sind Figur 1 zu entnehmen.

### Beispiel 5: Polykondensation von organischen Siliciumverbindungen in Gegenwart von immobilisierter Lipase

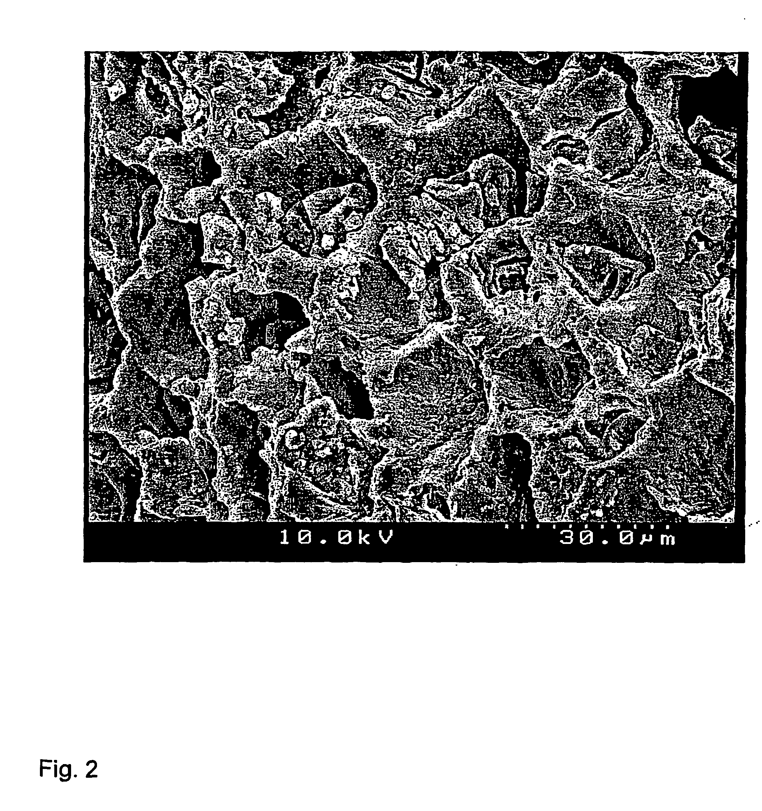

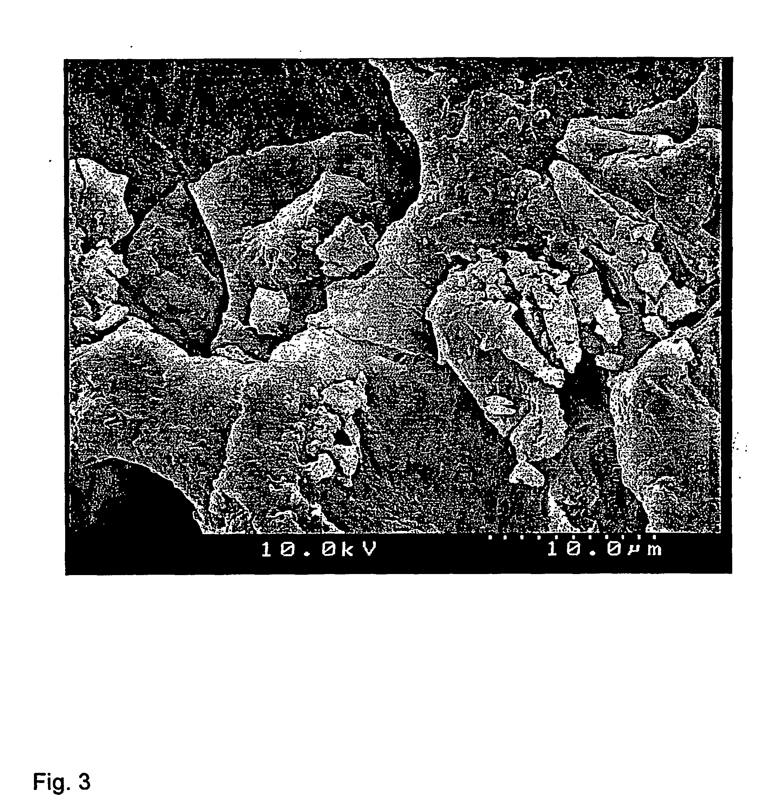

Zu auf Accurel 1001 immobilisierter Lipase in wäßriger Lösung wurde Tetrabutoxysilan gegeben und 3 Wochen bei Raumtemperatur stehen gelassen. Es bildeten sich kristalline Aufwachsungen auf dem Träger, und zwar an den Stellen, die mit Lipase belegt waren. In den Figuren 2 und 3 sind deutlich die kristallinen Aufwachsungen auf dem Träger zu erkennen.

**Patentansprüche**

1. Verfahren zur Polykondensation von organischen Siliciumverbindungen in Lösung bei pH-Werten zwischen 6 und 8 in Gegenwart eines Enzyms, **dadurch gekennzeichnet, daß** das Enzym eine Lipase ist.

2. Verfahren nach Anspruch 1, **dadurch gekennzeichnet, daß** die Lipase auf einem Träger immobilisiert ist.

3. Verfahren nach Anspruch 1 oder 2, **dadurch gekennzeichnet, daß** die Lösung Wasser und/oder eine Puffersubstanz enthält.

4. Verfahren nach Anspruch 2, **dadurch gekennzeichnet, daß** die Lösung ein organisches Lösungsmittel enthält.

5. Verfahren nach Anspruch 2, **dadurch gekennzeichnet, daß** das Trägermaterial Polypropylen, Polystyrol und/oder Polyurethanschaumstoff enthält.

6. Verfahren nach einem der Ansprüche 1 bis 5, **dadurch gekennzeichnet, daß** eine Lipase aus Pseudomonas-Arten eingesetzt wird.

7. Verfahren nach Anspruch 1, 3 oder 6, **dadurch gekennzeichnet, daß** das entstandene Polykondensationsprodukt von der Lipase abgetrennt und durch Dialyse gereinigt wird.

8. Verfahren nach einem der Ansprüche 1 bis 6, **dadurch gekennzeichnet, daß** die organische Siliciumverbindung ausgewählt ist aus der Gruppe aus

   - (RO)(R$^1$O)(R$^2$O)(R$^3$O)Si,
   - (RO)(R$^1$O)(R$^2$O)SiR$^3$,
   - (RO)(R$^1$O)Si(R$^2$)(R$^3$) und
   - (RO)SiR$^1$R$^2$R$^3$,

   mit R, R$^1$, R$^2$ und R$^3$, die unabhängig voneinander C$_1$- bis C$_{10}$-Alkyl, C$_3$- bis C$_{10}$-Cycloalkyl, C$_4$- bis C$_{20}$-Alkylcycloalkyl, Aryl, C$_6$- bis C$_{16}$-Alkylaryl sind, wobei die Alkylgruppen linear oder verzweigt sind.

**Claims**

1. A process for the polycondensation of organic silicon compounds in solution at from pH 6 to 8 in the presence of an enzyme, wherein the enzyme is a lipase.

2. A process as claimed in claim 1, wherein the lipase is immobilized on a carrier.

**3.** A process as claimed in claim 1 or 2, wherein the solution comprises water and/or a buffer substance.

**4.** A process as claimed in claim 2, wherein the solution comprises an organic solvent.

**5.** A process as claimed in claim 2, wherein the carrier material comprises polypropylene, polystyrene and/or polyurethane foam.

**6.** A process as claimed in one of claims 1 to 5, which comprises using a lipase from Pseudomonas species.

**7.** A process as claimed in claim 1, 3 or 6, which comprises removing the resulting polycondensation product from the lipase and purifying said product by dialysis.

**8.** A process as claimed in one of claims 1 to 6, wherein the organic silicon compound is selected from the group comprising

- $(RO)(R^1O)(R^2O)(R^3O)Si$,
- $(RO)(R^1O)(R^2O)SiR^3$,
- $(RO)(R^1O)Si(R^2)(R^3)$ and
- $(RO) SiR^1R^2R^3$,

where $R$, $R^1$, $R^2$ and $R^3$ are independently of one another $C_1$- to $C_{10}$-alkyl, $C_3$- to $C_{10}$-cycloalkyl, $C_4$- to $C_{20}$-alkylcycloalkyl, aryl, $C_6$- to $C_{16}$-alkylaryl, the alkyl groups being linear or branched.

## Revendications

**1.** Procédé de polycondensation de composés organiques de silicium en solution à des valeurs de pH comprises entre 6 et 8, en présence d'une enzyme, **caractérisé en ce que** l'enzyme est une lipase.

**2.** Procédé suivant la revendication 1, **caractérisé en ce que** la lipase est immobilisée sur un support.

**3.** Procédé suivant la revendication 1 ou 2, **caractérisé en ce que** la solution contient de l'eau et/ou une substance tampon.

**4.** Procédé suivant la revendication 2, **caractérisé en ce que** la solution contient un solvant organique.

**5.** Procédé suivant la revendication 2, **caractérisé en ce que** la matière de support contient du polypropylène, du polystyrène et/ou une mousse de polyuréthanne..

**6.** Procédé suivant l'une des revendications 1 à 5, **caractérisé en ce qu'**on met en oeuvre une lipase des espèces Pseudomonas.

**7.** Procédé suivant la revendication 1, 3 ou 6, **caractérisé en ce que** le produit de polycondensation obtenu est séparé de la lipase et purifié par dialyse.

**8.** Procédé suivant l'une des revendications 1 à 6, **caractérisé en ce que** le composé organique de silicium est choisi parmi le groupe

- $(RO)(R^1O)(R^2O)(R^3O)Si$,
- $(RO)(R^1O)(R^2O)SiR^3$,
- $(RO)(R^1O)Si (R^2)(R^3)$, et
- $(RO) SiR^1R^2R^3$,

où $R$, $R^1$, $R^2$ et $R^3$ représentent, indépendamment les uns des autres, un alkyle en $C_1$-$C_{10}$, cycloalkyle en $C_3$-$C_{10}$, alkylcycloalkyle en $C_4$-$C_{20}$, aryle, alkylaryle en $C_6$-$C_{16}$, les groupes alkyle étant linéaires ou ramifiés.

Fig. 1

Fig. 2

Fig. 3